# EUROPEAN PATENT APPLICATION

(11) **EP 1 000 595 A1**
(43) Date of publication of application: **17.05.2000**
(21) Application number: 99120095.7
(22) Date of filing: 21.10.1999
(51) Int. Cl.: A61F 2/46

(54) **Extractor for removing the bioceramic insert from the acetabular cup of a hip prosthesis**

(30) Priority: 11.11.1998 IT BO980622
(71) Applicant: Società per Azioni SAMO, 40057 Cadriano di Granarolo Emilia (Bologna) (IT)
(72) Inventor: Alfonsi, Stefano, 40131 Bologna (IT); Lippi, Andrea, 40012 Calderara di Reno (Bologna) (IT); Micelli, Teodora, 40138 Bologna (IT); Sandrini, Roberto, 40017 S. Giovanni in Persiceto (Bologna) (IT)
(74) Representative: Modiano, Guido, Dr.-Ing.

(57) **Abstract**

An extractor for removing the bioceramic insert (2) from the acetabular cup (3) of a hip prosthesis and an associated modified insert, wherein the insert (2) is axially provided with a through hole (4) and the extractor (1) comprises: a tubular shaft (5) which has, at one end, a rigidly coupled ring (9) of claws (10) which can spread out elastically, the ring (9) being adapted to rigidly couple in the hole (4) of the insert (2); a bush (11) which is externally frustum-shaped, has an internal thread (12) and can be inserted in the inlet of the ring (9) in order to spread out the claws (10); and a stem (14) which is inserted in the shaft (5) and can be screwed manually into the bush (11), the tip (14a) of the stem (14) being adapted, by screwing, to press against the end wall of the cup (3) and disengage it from the insert (2).

## Description

The present invention relates to an extractor for removing the bioceramic insert from the acetabular cup of a hip prosthesis and to the associated modified insert.

Conventional hip prostheses are constituted by an acetabular cup made of metallic material which has a frustum-shaped annular seat in which it is possible to couple, with an interlocking fit, the outer surface of an insert made of a material such as bioceramics, in which a spheroidal seat is formed for the articulation of the substantially spherical end of the head of the femur made of ceramic material.

The coupling of the bioceramic insert in the annular seat of the cup made of metallic material, so as to have a frustum-shaped surface against a frustum-shaped surface, is stable and is not subject to the onset of plays between the parts: the stability and solidity of the coupling, however, is a negative factor when it is necessary to remove the insert from the cup, for example in case of subsequent surgical procedures or of intraoperative malpositioning.

The aim of the present invention is to obviate the cited drawbacks of conventional devices, i.e., to provide an extractor for removing the bioceramic insert from the acetabular cup of a hip prosthesis and an associated modified insert whose resistance to static loads is greater than that of the corresponding unmodified insert, accordingly also increasing the safety of the device.

Within the scope of this aim, an object of the present invention is to provide a structure which is simple, relatively easy to provide in practice, safe in use, effective in operation and relatively low in cost.

This aim, this object and others which will become apparent hereinafter are achieved by the present extractor for removing the bioceramic insert from the acetabular cup of a hip prosthesis and by the associated modified insert, characterized in that the insert is axially provided with a through hole and in that said extractor comprises: a tubular shaft which has, at one end, a rigidly coupled ring of claws which can spread out elastically, said ring being adapted to rigidly couple in said hole of the insert; a bush which is externally frustum-shaped, has an internal thread and can be inserted in the inlet of said ring in order to spread out said claws; and a stem which is inserted in said shaft and can be screwed manually into said bush, the tip of said stem being adapted, by screwing, to press against the end wall of the cup and disengage it from the insert.

Further characteristics and advantages of the present invention will become apparent from the following detailed description of a preferred but not exclusive embodiment of an extractor for removing the bioceramic insert from the acetabular cup of a hip prosthesis and of an associated modified insert according to the invention, illustrated only by way of non-limitative example in the accompanying drawings, wherein:

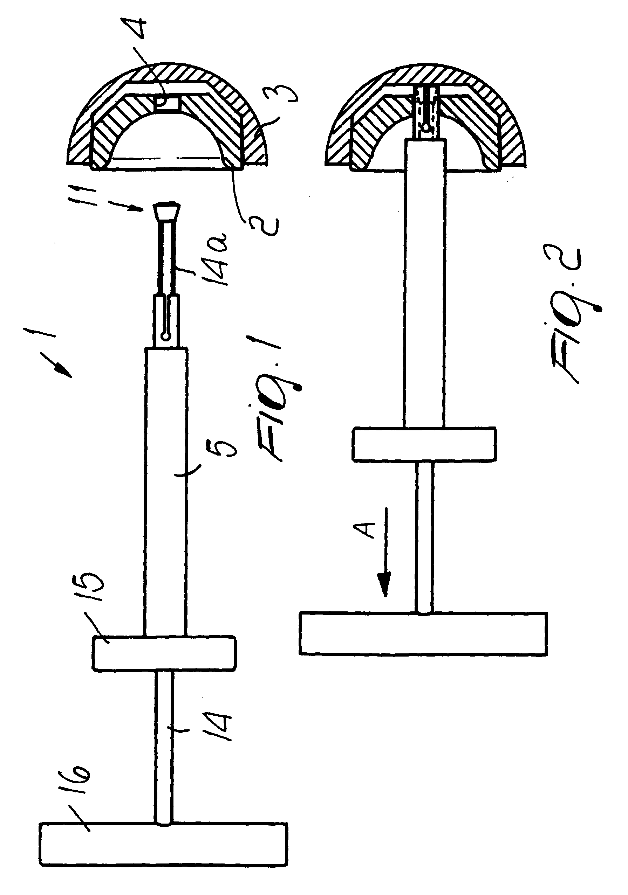

Figure 1 is a side view of an extractor in a configuration in which it is aligned with the acetabular cup;
Figure 2 is a side view of the extractor arranged in the cup;
Figure 3 is a sectional side view, taken along a diametrical plane, of the tubular shaft of the extractor;
Figure 4 is a sectional side view, taken along a diametrical plane, of the deformable ring of the tubular shaft;
Figure 5 is a front view of the ring of Figure 4;
Figure 6 is a sectional side view, taken along a diametrical plane, of the frustum-shaped bush of the extractor;

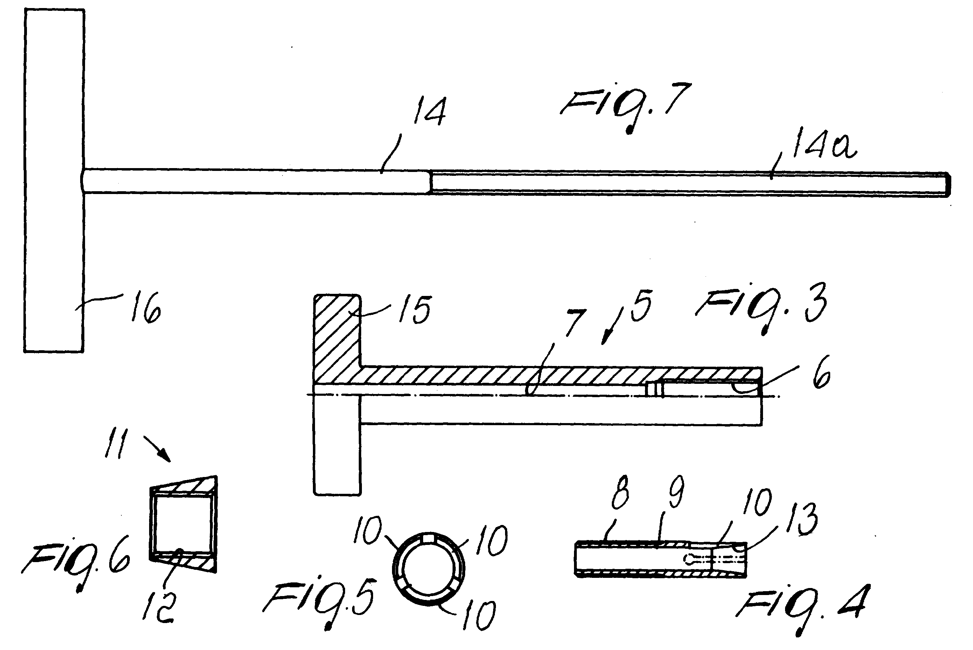

Figure 7 is a side view of the stem of the extractor according to the invention.

With particular reference to the above figures, the reference numeral 1 generally designates an extractor for removing the bioceramic insert 2 from the acetabular cup 3 of a hip prosthesis and the associated modified insert according to the invention.

The modified insert 2 is axially crossed by a through hole 4 which has a smaller diameter and preferably has a frustum-like shape which has been found in practice to increase the static load resistance of said insert.

The extractor comprises a tubular shaft 5 in which an initial portion 6 of the through hole 7 is threaded for the screwing of the threaded end 8 of a ring 9 of three elastically openable claws 10, which is adapted to rigidly couple inside the hole 4 of the insert: the claws 10 are obtained by means of longitudinal notches in the surface of the ring.

The reference numeral 11 designates a bush which has a slightly frustum-shaped outer surface and an internal thread 12 which can be inserted in the inlet of the ring 9 in order to spread out the claws 10: the internal hole 13 of the ring of claws 10 has a slightly diverging frustum-like shape in order to facilitate the insertion of the converging end of the bush 11.

A stem 14 is inserted in the shaft 5 and is provided with a threaded end 14a for manual screwing into the bush 11; the tip of said stem is meant to press, by means of said screwing action, against the end wall of the cup 3 (acting against the bush 11, which pushes the claws radially so as to grip the insert 2) and to disengage it from the insert 2.

The handling ends of the tubular shaft 5 and of the stem 14 have respective handpieces 15 and 16 which are rigidly coupled thereto and are constituted by cross-members arranged substantially in a T-shaped configuration with respect to the shaft and the stem.

The operation of the extractor according to the invention is as follows: the ring of claws 10 is inserted in the hole 4 of the insert, and while the shaft 5 is held stationary, the handpiece 16 is moved away from the handpiece 15, as shown by the arrow A, producing the radial spacing of the claws 10 and the coupling of the ring in the hole 4 of the insert; by screwing the handpiece 16, the bush is made to exit from the tip of the stem 14, which presses against the end wall of the cup 3: as the handpiece 16 is screwed in further with respect to the handpiece 15, the insert is forced to disengage from the cup; by moving the handpiece 16 toward the handpiece 15, the claws retract radially and it is possible to release the extractor from the insert.

It has thus been observed that the invention achieves the intended aim and object.

The invention thus conceived is susceptible of numerous modifications and variations, all of which are within the scope of the inventive concept.

All the details may also be replaced with other technically equivalent elements.

In practice, the materials employed, as well as the shapes and the dimensions, may be any according to requirements without thereby abandoning the scope of the protection of the appended claims.

The disclosures in Italian Patent Application No. BO98A000622 from which this application claims priority are incorporated herein by reference.

Where technical features mentioned in any claim are followed by reference signs, those reference signs have been included for the sole purpose of increasing the intelligibility of the claims and accordingly, such reference signs do not have any limiting effect on the interpretation of each element identified by way of example by such reference signs.

## Claims

1. An extractor for removing the bioceramic insert (2) from the acetabular cup (3) of a hip prosthesis and an associated modified insert, characterized in that the insert (2) is axially provided with a through hole (4) and in that said extractor (1) comprises: a tubular shaft (5) which has, at one end, a rigidly coupled ring (9) of claws (10) which can spread out elastically, said ring (9) being adapted to rigidly couple in said hole (4) of the insert (2); a bush (11) which is externally frustum-shaped, has an internal thread (12) and can be inserted in the inlet of said ring (9) in order to spread out said claws (10); and a stem (14) which is inserted in said shaft (5) and can be screwed manually into said bush (11), the tip (14a) of said stem (14) being adapted, by screwing, to press against the end wall of the cup (3) and disengage it from the insert (2).

2. The extractor according to claim 1, characterized in that said ring (9) is rigidly coupled to said tubular shaft (5) by screwing its threaded end (8) into the threaded initial portion (6) of the hole (7) of said shaft (5).

3. The extractor according to one or more of the preceding claims, characterized in that the internal hole (13) of said ring (9) of claws (10) diverges slightly so as to facilitate the insertion of the converging end of said bush (11).

4. The extractor according to one or more of the preceding claims, characterized in that the actuation ends of said tubular shaft (5) and of said stem (14) have respective handpieces (15, 16) rigidly coupled thereto.

5. The extractor according to one or more of the preceding claims, characterized in that said handpieces (15, 16) are arranged substantially in a T-shaped configuration with respect to the shaft (5) and the stem (14).

6. The extractor according to one or more of the preceding claims, characterized in that said hole (4) of the modified insert (2) has a smaller diameter, is preferably frustum-shaped and is adapted to increase the static load resistance of said insert (2).
